# EUROPEAN PATENT APPLICATION

(11) **EP 4 640 132 A1**
(43) Date of publication of application: **29.10.2025**
(21) Application number: 25168819.8
(22) Date of filing: 07.04.2025
(51) Int. Cl.: A61B 1/00, A61B 1/247, A61B 5/00, A61C 9/00, A61C 19/04, G06T 3/4038

(54) **METHOD AND DEVICE FOR ADJUSTING FIELD OF VIEW FOR INTRAORAL SCANNERS**

(30) Priority: 26.04.2024 CN 202410518909
(71) Applicant: Shanghai Alliedstar Medical Technology Co., Ltd., Shanghai 201200 (CN)
(72) Inventor: WU, Houhang, Shanghai, 201200 (CN); CAO, Boyang, Shanghai, 201200 (CN); XIONG, Jingqi, Shanghai, 201200 (CN)
(74) Representative: Winter, Brandl - Partnerschaft mbB

(57) **Abstract**

A method is provided. The method includes collecting surface information of teeth through a sensor and a tip of an intraoral scanner. The method also includes detecting an overlap between a first area and a second area based on the surface information. The first area indicates a field of view of the sensor through the tip for collecting the surface information. The second area indicates a field of view of the tip for collecting the surface information. The method further includes adjusting the first area relative to the second area. A device implementing the method is also disclosed.

## Description

### TECHNOLOGICAL FIELD

The present disclosure generally relates to intraoral scanners, and in particular, to a method and device for adjusting field of view for intraoral scanners based on image processing.

### BACKGROUND

An intraoral scanner includes a tip that may be inserted into a patient's mouth to collect the information or data of oral cavity or teeth. If the size of the tip is smaller, the patient may feel more comfortable with using the intraoral scanner. Thus, it is desired to control or reduce the size of the tip. On the other hand, a larger size of the tip may be more beneficial for registration of the full mouth three-dimensional (3D) data. A tradeoff may need to be considered.

Therefore, it would be desirable to have a solution that takes into account at least some of the issues discussed above, as well as other possible issues.

### BRIEF SUMMARY

Example embodiments of the present disclosure relates to a method and device for adjusting field of view for intraoral scanners based on image processing. The present disclosure includes, without limitation, the following example embodiments.

Some embodiments provide a method. The method comprises collecting surface information of teeth through a sensor and a tip of an intraoral scanner; detecting an overlap between a first area and a second area based on the surface information, wherein the first area indicates a field of view of the sensor through the tip for collecting the surface information, and wherein the second area indicates a field of view of the tip for collecting the surface information; and adjusting the first area relative to the second area.

In some embodiments of the method, adjusting the first area includes cropping the first area to maintain the overlap between the first area and the second area.

In some embodiments of the method, adjusting the first area includes moving the first area to increase the overlap between the first area and the second area.

In some embodiments of the method, moving the first area includes moving the first area entirely into the second area.

In some embodiments of the method, moving the first area includes moving a center of the field of view of the sensor.

In some embodiments of the method, detecting the overlap includes detecting whether the first area is at least partially outside the second area using a machine learning model.

Some embodiments provide a device. The device comprises a processor and a memory storing executable instructions that, in response to execution by the processor, cause the device to at least perform the method of any preceding embodiments, or any combination of thereof.

Some embodiments provide a non-transitory computer-readable storage medium comprising executable instructions stored therein that, in response to execution by a processor of a device, causes the device to at least perform the method of any preceding example embodiments, or any combination thereof.

Some embodiments provide an apparatus. The apparatus comprises means for collecting surface information of teeth through a sensor and a tip of an intraoral scanner; means for detecting an overlap between a first area and a second area based on the surface information, wherein the first area indicates a field of view of the sensor through the tip for collecting the surface information, and wherein the second area indicates a field of view of the tip for collecting the surface information; and means for adjusting the first area relative to the second area.

These and other features, aspects, and advantages of the present disclosure will be apparent from a reading of the following detailed description together with the accompanying figures, which are briefly described below. The present disclosure includes any combination of two, three, four or more features or elements set forth in this disclosure, regardless of whether such features or elements are expressly combined or otherwise recited in a specific embodiment described herein. This disclosure is intended to be read holistically such that any separable features or elements of the disclosure, in any of its aspects and embodiments, should be viewed as combinable unless the context of the disclosure clearly dictates otherwise.

It will therefore be appreciated that this Brief Summary is provided merely for purposes of summarizing some embodiments so as to provide a basic understanding of some aspects of the disclosure. Other embodiments, aspects and advantages will become apparent from the following detailed description taken in conjunction with the accompanying figures which illustrate, by way of example, the principles of some described embodiments.

### BRIEF DESCRIPTION OF THE FIGURE(S)

Having thus described embodiments of the disclosure in general terms, reference will now be made to the accompanying figures, which are not necessarily drawn to scale, and wherein:
FIGs. 1A and 1B illustrate an intraoral scanner, according to some embodiments of the present disclosure;
FIGs. 2A and 2B illustrate a first area and a second area based on collected surface information of teeth, according to some embodiments of the present disclosure;
FIGs. 3A and 3B illustrate adjusting a first area relative to a second area, according to some embodiments of the present disclosure;
FIGs. 4A and 4B illustrate adjusting a first area based on image processing, according to some other embodiments of the present disclosure;
FIG. 5 illustrates a method of adjusting field of view for an intraoral scanner, according to some embodiments of the present disclosure; and
FIG. 6 illustrates a device for adjusting field of view for an intraoral scanner, according to some embodiments.

### DETAILED DESCRIPTION

Some embodiments of the present disclosure will now be described more fully hereinafter with reference to the accompanying figures, in which some, but not all embodiments of the disclosure are shown. Indeed, various embodiments of the disclosure may be embodied in many different forms and should not be construed as limited to the embodiments set forth herein; rather, these embodiments are provided so that this disclosure will be thorough and complete, and will fully convey the scope of the disclosure to those skilled in the art. For example, unless otherwise indicated, reference something as being a first, second or the like should not be construed to imply a particular order. Like reference numerals refer to like elements throughout.

The present disclosure discloses an automated field of view adjustment method based on image processing for intraoral scanners. An intraoral scanner may include a sensor and a tip for collecting information or data of oral cavity or teeth. The method may utilize image processing techniques to identify an effective field of view of the sensor. The effective field of view may be the overlap between the sensor's field of view and the tip's field of view. The method may adjust the sensor's field of view to maintain or increase or maximize the effective field of view. By doing so, when designing or manufacturing the intraoral scanners, the tip's field of view may be reduced and hence the size of the tip may be reduced.

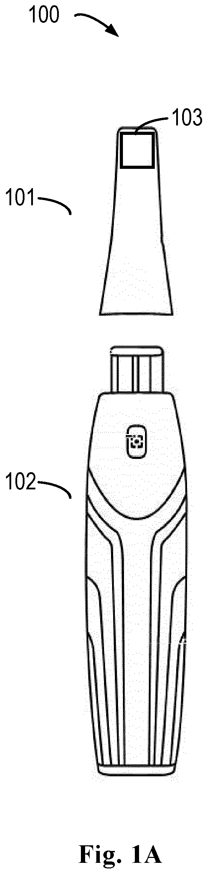

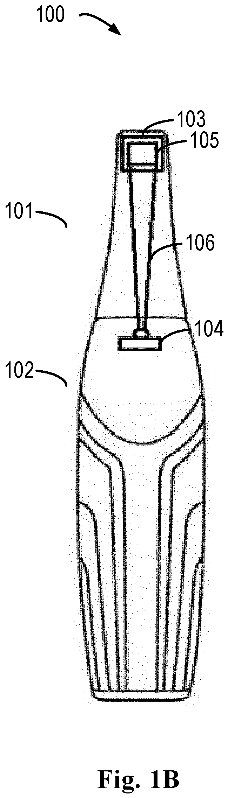

FIGs. 1A and 1B illustrate an intraoral scanner 100, according to some embodiments of the present disclosure. As shown in FIG. 1A, the intraoral scanner 100 may include a tip 101 and a body 102. The tip 101 may be a pluggable part or may be integrated with the intraoral scanner 100. The tip 101 may have a field of view 103. As shown in FIG. 1A, the tip's field of view 103 may be formed through an open area at or close to the end of the tip 101. The tip's field of view 103 may be determined by hardware of the tip 101. For an intraoral scanner 100, the tip's field of view 103 may be a fixed field of view, which may have the same size as the open area at or close to the end of the tip 101. The tip's field of view 103 may be used for collecting information or data of oral cavity or teeth, e.g., surface information of the teeth.

As shown in FIG. 1B, the intraoral scanner 100 may include a sensor 104. In one example, the sensor 104 may be included in or installed on the body 102. In another example, the sensor 104 may be included in or installed on the tip 101, e.g., installed close to the open area at or close to the end of the tip 101. In one example, the sensor 104 may be a camera. The sensor 104 may have a field of view 105. As shown in FIG. 1B, there can be an optical path 106 from the sensor 104 to the tip 101 or through the tip's field of view 103. The sensor's field of view 105 may be an outward field of view along the optical path 106 through the tip 101 or through the tip's field of view 103.

In some embodiments, as shown in FIG. 1B, the sensor's field of view 105 may be formed through the open area at or close to the end of the tip 101. In one example, the sensor's field of view 105 may be smaller than the tip's field of view 103, and may be entirely included in the tip's field of view 103. In another example, the sensor's field of view 105 may be at least partially outside the tip's field of view 103. In a further example, the sensor's field of view 105 may be larger than the tip's field of view 103. The tip's field of view 105 may be used for collecting information or data of oral cavity or teeth, e.g., surface information of the teeth. The tip 101 can be inserted into a patient's oral cavity such that the sensor 104 can collect information or data of oral cavity or teeth.

In some embodiments, a computing device may collect surface information of teeth through the sensor 104 and the tip 101 of the intraoral scanner 100. The computing device may be separate from the intraoral scanner 100 and may communicate with the intraoral scanner 100 through wired or wireless communications. In some other embodiments, the computing device may be the intraoral scanner 100. The computing device may be the device 600 as described below.

In some embodiments, the surface information of teeth may include two-dimensional (2D) image information of the teeth surface, or three-dimensional (3D) image information including depth information, or other feature information. In some embodiments, the surface information may be collected by the sensor and transmitted to the computing device. In some embodiments, the surface information may be collected by the sensor based on photoelectric conversion using technologies such as confocal, structured light, binocular vision and other technologies, as understood in the art.

Taking structured light technology as an example, the pre-designed coded stripe pattern can be projected onto the surface of the teeth through a projection device such as a digital light processing (DLP) display. The two-dimensional image information of the teeth surface superimposed with the stripe pattern can be obtained through an image sensor with a photosensitive array. A series of image processing methods can be used to obtain the stripe coordinate information in the stripe image, and the projected stripes and the image stripes collected by the sensor can be mapped and matched. The three-dimensional coordinate information of the measured teeth surface can be calculated based on the trigonometric principle or the spatial geometry principle.

In some embodiments, the computing device may detect an overlap between a first area and a second area based on the surface information. The first area indicates the field of view 105 of the sensor 104 through the tip 101 for collecting the surface information, and the second area indicates the field of view 103 of the tip 101 for collecting the surface information.

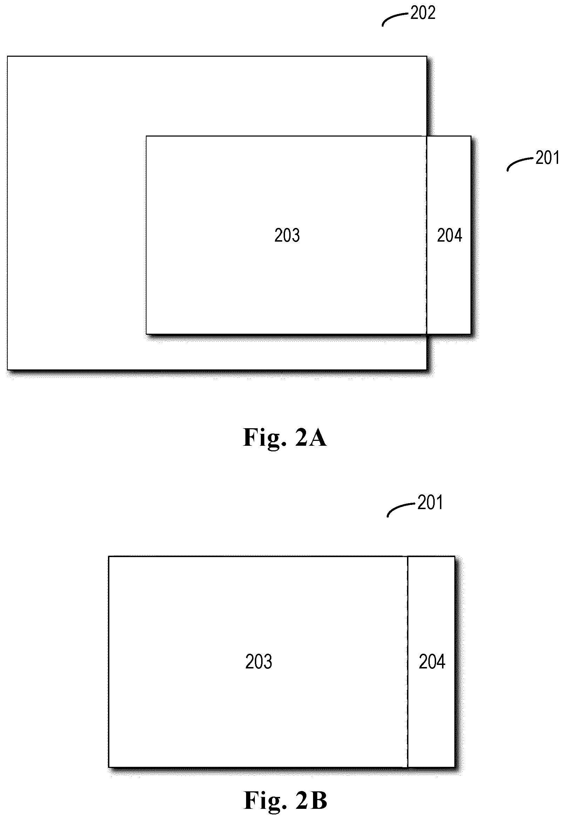

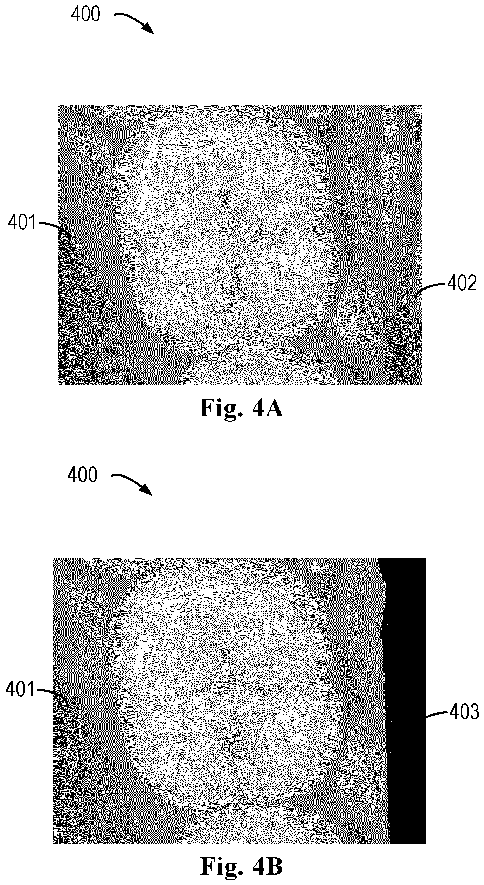

FIGs. 2A and 2B illustrate a first area and a second area based on collected surface information of teeth, according to some embodiments of the present disclosure. As shown in FIG. 2A, the first area 201 indicates the sensor's field of view 105, and the second area 202 indicates the tip's field of view 103. The overlapping area 203 may indicate the effective field of view of the sensor 104. The effective field of view of the sensor 104 may be the overlap between the sensor's field of view 105 and the tip's field of view 103. The non-overlapping area 204 indicates the part that the first area 201 outside the second area 202, which may be an invalid field of view of the sensor 104 when using the intraoral scanner 100. The overlapping area 203 and the non-overlapping area 204 may be segmented by the line between using image processing as described below. The line may be a straight line as shown in FIG. 2B, but also may be a polyline or a curved line as shown in FIG. 4B below.

The second area 202 is for illustration purpose only in the present disclosure. After collecting the surface information of the teeth, the computing device may not generate and display an image of the second area 202 in the computing device. The second area 202 or the tip's field of view 103 may be real field of view. The computing device may have information such as the size and shape of the second area 202 or the tip's field of view 103 based on parameters of the tip 101 or the intraoral scanner 100, without the need to generate and display an image of the second area 202 for image processing.

Based on the collected surface information of the teeth, the computing device may generate and display an image of the first area 201 in the computing device for further image processing. As shown in FIG. 2B, the first area 201, the overlapping area 203 and the non-overlapping area 204 may be generated and displayed as one or more images in the computing device.

In some embodiments, to detect the overlap between the first area 201 and the second area 202, the computing device may first detect whether the first area 201 is at least partially outside the second area 202 using a machine learning model. The computing device may detect the overlapping area 203 and/or the non-overlapping area 204 using a machine learning model. For example, the computing device can use deep learning methods to pre-train images, generate prediction models, and use AI reasoning to identify the effective and/or invalid sensor's field of view range in the current field of view.

In some embodiments, the computing device may build a multi-task classification and segmentation model to realize the automatic detection and segmentation of the invalid field of view of the sensor 104, i.e., the non-overlapping area 204. The classification task is used to detect whether the image of the first area 201 contains the invalid field of view, and the segmentation task is used to segment the invalid field of view from the image of the first area 201.

In some embodiments, building the multi-task classification and segmentation model includes the following steps. The first step is data collection and labeling. A computing device may collect images of sensor's field of view using an intraoral scanner. A person may manually mark or label images indicating normal sensor's field of view (that is, images that do not contain invalid sensor's field of view) as positive samples. Similarly, a person may perform manual annotation or labelling to images whose field of view exceeds the tip's field of view (that is, images that contain invalid sensor's field of view) as negative samples. This can create a dataset for deep learning training.

In some embodiments, the second step is data preprocessing. The data set created in the first step can be divided into training set and test set according to a ratio, such as a ratio of 7:3. Appropriate data enhancement can be performed on each image in the training set. The enhancement operations may include image cropping and combination, flipping, offset, rotation, brightness change, and adding noise and so on.

In some embodiments, the third step is model construction and training. To build the multi-task classification and segmentation model, a computing device can use Unet as the backbone network. Unet contains two parts: an encoder and a decoder. The encoder is used for feature extraction, followed by a classification head layer for classifying whether an image contains an invalid sensor's field of view. The decoder is followed by a segmentation head layer to predict the invalid field of view range based on the extracted feature information. The model includes two tasks: classification and segmentation. The total loss function includes classification loss and segmentation loss. The classification loss uses the Focal Loss loss function to reduce the impact on detection results due to imbalance of positive and negative samples. The segmentation loss uses cross-entropy loss. The training of the model uses the Adam algorithm to optimize the loss function. The model can be trained using the training set, and the parameters can be adjusted based on the model generalization performance on the test set, and finally the optimal detection model or the multi-task classification and segmentation model can be obtained.

In some embodiments, the fourth step is prediction of invalid sensor's field of view. For a frame of scanned image to be predicted, image data of 640 × 480 size can be obtained after preprocessing, and the image is input into the detection model. After forward propagation, the model may output 2 results, including the image classification result (that is, whether the image contains an invalid sensor's field of view) and segmentation result as shown in FIG. 2B. For scanned data or an image containing an invalid field of view, the invalid field of view range can be obtained based on the segmentation results. Then the invalid field of view area in the image can be eliminated or cropped, leaving the remaining effective field of view area in the image.

In some embodiments, the computing device may also detect the overlapping area 203 and/or the non-overlapping area 204 using methods including brightness adjustment, contrast adjustment, edge extraction, frequency domain conversion and so on.

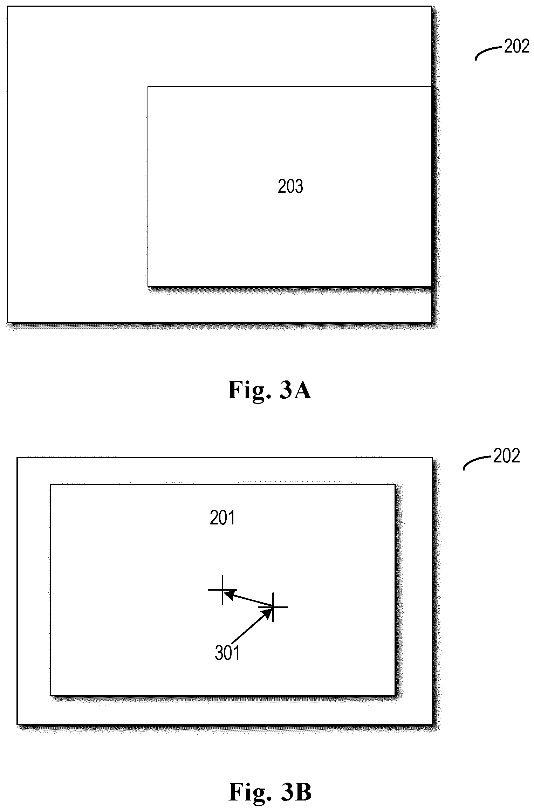

FIGs. 3A and 3B illustrate adjusting a first area relative to a second area, according to some embodiments of the present disclosure. In some embodiments, the computing device may adjust the first area 201 by cropping the first area to maintain the overlap 203 between the first area 201 and the second area 202. As shown in FIG. 3A, for the first area 201 including the overlapping area 203 and the non-overlapping area 204, the non-overlapping area 204 has been cropped. And only the overlapping area 203 is maintained. The computing device may not generate and display an image of the second area 202 in the computing device, but may generate and display an image of the first area 201 in the computing device for cropping.

In some embodiments, the computing device may adjust the first area 201 by moving the first area 201 to increase the overlapping area 203 between the first area 201 and the second area 202. In some embodiments, the computing device may move the first area 201 entirely into the second area. As shown in FIG. 3B, the first area 201 can be moved entirely into the second area 202 to increase or maximize the overlapping area 203. The overlapping area 203 in these embodiments is equal to the first area 201. That is, the sensor's field of view 105 is equal to the effective field of view of the sensor 104. The computing device may not generate and display an image of the second area 202 in the computing device, but may generate and display an image of the first area 201 in the computing device for moving.

In some embodiments, as shown in FIG. 3B, the computing device may move the first area 201 by moving a center 301 of the sensor's field of view 105. In one example, moving the center 301 of the sensor's field of view 105 may be realized by moving the physical position of the sensor 104 through some mechanical devices. In another example, moving the center 301 of the sensor's field of view 105 may be realized by modifying the starting position of the photosensitive area supported within the sensor 104.

FIGs. 4A and 4B illustrate adjusting a first area based on image processing, according to some embodiments of the present disclosure. As shown in FIG. 4A, the computing device can generate and display image 400 based on the collected surface information of teeth as described above. The image 400 may correspond to the first area 201 indicating the sensor's field of view 105. The part 401 may correspond to the overlapping area 203 indicating the effective field of view of the sensor 104. The part 402 may correspond to the non-overlapping area 204 indicating the invalid field of view of the sensor 104. As shown in FIG. 4B, the computing device can use a black mask to invalidate the data of the part 402. As described above, the computing device can also crop the part 402 from the image 400.

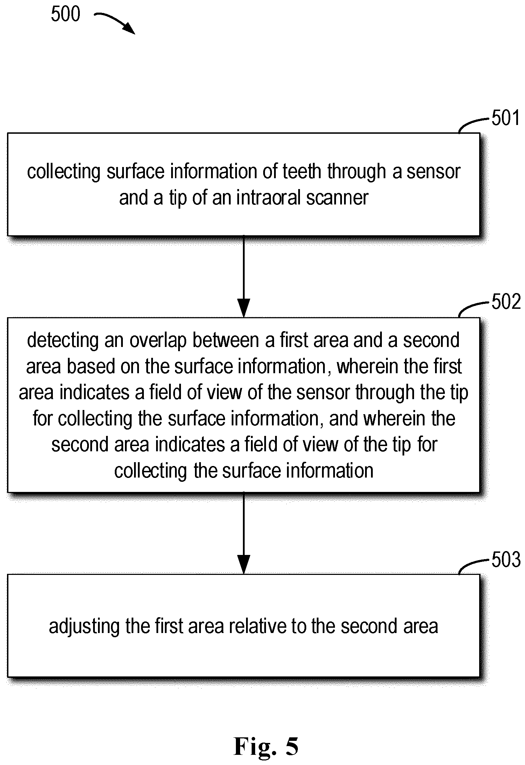

FIG. 5 illustrates a method 500 of adjusting field of view for an intraoral scanner, according to some embodiments of the present disclosure. As shown, at block 501, the method includes collecting surface information of teeth through a sensor and a tip of an intraoral scanner. At block 502, the method includes detecting an overlap between a first area and a second area based on the surface information, wherein the first area indicates a field of view of the sensor through the tip for collecting the surface information, and wherein the second area indicates a field of view of the tip for collecting the surface information. At block 503, the method includes adjusting the first area relative to the second area.

In some embodiments, an apparatus capable of performing the method 500 (for example, the device or apparatus 600 as described below) may comprise means for performing the respective steps of the method 500. The means may be implemented in any suitable form. For example, the means may be implemented in a circuitry or software module.

In some embodiments, the apparatus comprises means for collecting surface information of teeth through a sensor and a tip of an intraoral scanner; means for detecting an overlap between a first area and a second area based on the surface information, wherein the first area indicates a field of view of the sensor through the tip for collecting the surface information, and wherein the second area indicates a field of view of the tip for collecting the surface information; and means for adjusting the first area relative to the second area.

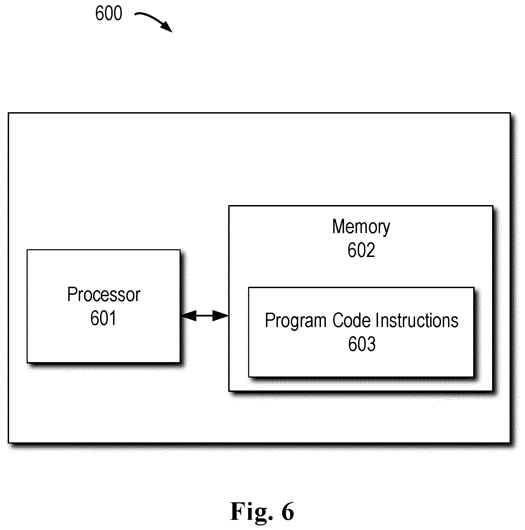

FIG. 6 illustrates a device or apparatus 600 for adjusting field of view for an intraoral scanner, according to some embodiments. The device 600 may implement the method 500. In some embodiments, the device 600 may be a computing device separate from the intraoral scanner 100. In some other embodiments, the device 600 may be the intraoral scanner 100.

As shown in FIG. 6, in some embodiments, the computing device 600 includes a processor 601 and a memory 602 coupled to the processor 601. In some examples, the processor 601 may itself include the memory 602.

In some examples, the processor 601 may be a microprocessor or microcontroller unit (MCU). The processor 601 may be composed of one or more processors alone or in combination with one or more memories. The processor is generally any piece of computer hardware that is capable of processing information such as, for example, data, computer programs and/or other suitable electronic information. The processor is composed of a collection of electronic circuits some of which may be packaged as an integrated circuit or multiple interconnected integrated circuits (an integrated circuit at times more commonly referred to as a "chip"). The processor may be configured to execute computer programs, which may be stored onboard the processor or otherwise stored in the memory 602 (of the same or another apparatus).

The processor 601 may be a number of processors, a multi-core processor or some other type of processor, depending on the particular implementation. Further, the processor may be implemented using a number of heterogeneous processor systems in which a main processor is present with one or more secondary processors on a single chip. Although the processor may be capable of executing a computer program to perform one or more functions, the processor of various examples may be capable of performing one or more functions without the aid of a computer program. In either instance, the processor may be appropriately programmed to perform functions or operations according to embodiments of the present disclosure.

In some examples, the memory 602 may be a computer-readable storage medium. The computer-readable storage medium is a non-transitory device capable of storing information, and is distinguishable from computer-readable transmission media such as electronic transitory signals capable of carrying information from one location to another. The memory 602 is generally any piece of computer hardware that is capable of storing information such as, for example, data, computer programs (e.g., computer-readable program code instructions 603) and/or other suitable information either on a temporary basis and/or a permanent basis. The memory may include volatile and/or nonvolatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above.

In some embodiments, the memory 602 stores computer-readable program code instructions 603. The processor 601 is configured to execute computer-readable program code instructions 603 stored in the memory 602. Execution of the program code instructions may produce a computer-implemented process such that the instructions executed by the computer, processor or other programmable apparatus provide operations for implementing functions described herein. Execution of instructions by a processor, or storage of instructions in a computer-readable storage medium, supports combinations of operations for performing the specified functions described herein. It will also be understood that one or more functions, and combinations of functions, may be implemented by special purpose hardware-based computer systems and/or processors which perform the specified functions, or combinations of special purpose hardware and program code instructions.

In some embodiments, the processor 601 is configured to execute computer-readable program code instructions 603 stored in the memory 602, such that the computing device 600 can be caused to implement the method 500.

In addition to the memory 602, the processor 601 may also be connected to one or more interfaces for displaying, transmitting and/or receiving information. The interfaces may include a communications interface (e.g., communications unit) and/or one or more user interfaces. The communications interface may be configured to transmit and/or receive information, such as to and/or from other apparatus(es), network(s) or the like. The communications interface may be configured to transmit and/or receive information by physical (wired) and/or wireless communications links. Examples of suitable communication interfaces include a network interface controller (NIC), wireless NIC (WNIC) or the like.

The user interfaces may include a display and/or one or more user input interfaces (e.g., input/output unit). The display may be configured to present or otherwise display information to a user, suitable examples of which include a liquid crystal display (LCD), light-emitting diode display (LED), plasma display panel (PDP) or the like. The user input interfaces may be wired or wireless, and may be configured to receive information from a user into the apparatus, such as for processing, storage and/or display. Suitable examples of user input interfaces include a microphone, image or video capture device, keyboard or keypad, touch-sensitive surface (separate from or integrated into a touchscreen), biometric sensor or the like. The user interfaces may further include one or more interfaces for communicating with peripherals such as printers, scanners or the like.

Many modifications and other embodiments of the disclosure set forth herein will come to mind to one skilled in the art to which the disclosure pertains having the benefit of the teachings presented in the foregoing description and the associated figures. Therefore, it is to be understood that the disclosure is not to be limited to the specific embodiments disclosed and that modifications and other embodiments are intended to be included within the scope of the appended claims. Moreover, although the foregoing description and the associated figures describe embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A method, comprising:
collecting surface information of teeth through a sensor and a tip of an intraoral scanner;
detecting an overlap between a first area and a second area based on the surface information, wherein the first area indicates a field of view of the sensor through the tip for collecting the surface information, and wherein the second area indicates a field of view of the tip for collecting the surface information; and
adjusting the first area relative to the second area.

2. The method of claim 1, wherein adjusting the first area includes cropping the first area to maintain the overlap between the first area and the second area.

3. The method of claim 1, wherein adjusting the first area includes moving the first area to increase the overlap between the first area and the second area.

4. The method of claim 3, wherein moving the first area includes moving the first area entirely into the second area.

5. The method of claim 3, wherein moving the first area includes moving a center of the field of view of the sensor.

6. The method of any preceding claim, wherein detecting the overlap includes detecting whether the first area is at least partially outside the second area using a machine learning model.

7. A device, comprising:
a processor; and
a memory storing executable instructions that, in response to execution by the processor, cause the device to at least implement the method according to any of claims 1 to 6.

8. A computer-readable storage medium comprising executable instructions stored therein that, in response to execution by a processor of a device, causes the device to at least implement the method according to any of claims 1 to 6.

9. A computer program comprising instructions which, when executed by a computer, cause the computer to at least implement the method according to any of claims 1 to 6.
